(19) Europäisches Patentamt
European Patent Office
Office européen des brevets

(11) Veröffentlichungsnummer: **0 054 218 B1**

(12) # EUROPÄISCHE PATENTSCHRIFT

(45) Veröffentlichungstag der Patentschrift: **03.10.84**

(51) Int. Cl.³: **C 07 C 125/065**, C 07 C 50/24

(21) Anmeldenummer: **81110031.2**

(22) Anmeldetag: **01.12.81**

(54) **Verfahren zur Herstellung von Urethanen.**

(30) Priorität: **12.12.80 DE 3046982**

(43) Veröffentlichungstag der Anmeldung: **23.06.82 Patentblatt 82/25**

(45) Bekanntmachung des Hinweises auf die Patenterteilung: **03.10.84 Patentblatt 84/40**

(84) Benannte Vertragsstaaten: **BE DE FR GB IT**

(56) Entgegenhaltungen:
**EP - A - 0 016 346**
**EP - A - 0 016 948**
**EP - A - 0 016 949**
**DE - A - 2 808 980**
**DE - A - 2 838 754**
**US - A - 4 266 070**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Stammann, Günter, Dr., Silesiusstrasse 78, *D-5000 Köln 80 (DE)***
Erfinder: **Becker, Robert, Dr., Martin-Heidegger-Strasse 8, D-5090 Leverkusen 3 (DE)**
Erfinder: **Grolig, Johann, Dr., Heinrich-Lübke-Strasse 22, D-5090 Leverkusen 1 (DE)**
Erfinder: **Waldmann, Helmut, Dr., Henry-T.-von-Böttinger-Strasse 15, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur Herstellung von Urethanen (Carbamidsäureestern oder Carbamaten) durch Umsetzung von primären Aminen mit organischen Hydroxylverbindungen und Kohlenmonoxid in Gegenwart eines Oxidationsmittels und in Gegenwart eines mindestens ein Edelmetall oder mindestens eine Edelmetallverbindung aufweisenden Katalysatorsystems, wobei man die Umsetzung in Gegenwart von chinoiden Verbindungen oder von in chinoide Verbindungen überführbaren Verbindungen durchführt.

Organische Isocyanate werden grosstechnisch im allgemeinen durch Umsetzung der entsprechenden Amine mit Phosgen hergestellt. Wegen des hohen Chlorbedarfs und der damit verbundenen hohen Energiekosten für die Phosgenherstellung ist man seit geraumer Zeit bestrebt, einen grosstechnisch gangbaren Weg zu organischen Isocyanaten aufzufinden, bei welchen sich die Verwendung von Phosgen erübrigt. Ein derartiger Weg besteht in der Umsetzung von primären Aminen mit Kohlenmonoxid und organischen Hydroxylverbindungen und einem Oxidationsmittel wie Luft oder organischer Nitroverbindung zu den entsprechenden Urethanen und in deren anschliessenden Spaltung in Isocyanat und Hydroxylgruppen aufweisende Verbindungen. Dieses phosgenfreie Herstellungsverfahren für Urethane wird beispielsweise in der DE-OS 2 910 132 oder in der DE-OS 2 908 251 (= EP-OS 0 016 346 oder US-Anmeldung 125 394 vom 27.2.1980) beschrieben. Beim Verfahren der letztgenannten Vorveröffentlichung werden primäre Amine durch Umsetzung mit Kohlenmonoxid, organischen Hydroxylverbindungen und molekularem Sauerstoff bzw. Nitroverbindungen als Oxidationsmittel katalytisch oxycarbonyliert, wobei neben Edelmetallkatalysatoren vorzugsweise 1 bis 5 Gew.-%, bezogen auf Gesamtgemisch, an chloridhaltigen, grösstenteils im Reaktionsgemisch unlöslichen anorganischen Feststoffen als Cokatalysatoren mitverwendet werden. Wegen des relativ hohen Gehalts der Systeme der genannten Vorveröffentlichung an chloridhaltigen Verbindungen können bei der Durchführung des vorveröffentlichten Verfahrens Korrosionsprobleme auftreten; ausserdem ist in der weitgehenden Unlöslichkeit der anorganischen Katalysatorkomponenten ein die grosstechnische Durchführbarkeit des bekannten Verfahrens beeinträchtigender Nachteil zu sehen.

Es war daher die Aufgabe der Erfindung, ein verbessertes Verfahren zur Herstelung von Urethanen aus den genannten Ausgangsmaterialien zur Verfügung zu stellen, bei welchem auf die Verwendung von weitgehend unlöslichen bzw. von korrodierend wirkenden Katalysatorkomponenten verzichtet werden kann, bzw. bei welchem der Anteil derartiger Komponenten im Reaktionsgemisch ganz wesentlich reduziert werden kann.

Diese Aufgabe konnte durch das nachstehend näher beschriebene erfindungsgemässe Verfahren gelöst werden.

Gegenstand der Erfindung ist ein Verfahren zur Herstellung von Urethanen durch Umsetzung von

a) primären Aminen mit
b) mindestens eine Hydroxylgruppe enthaltenden organischen Verbindungen und
c) Kohlenmonoxid in Gegenwart von
d) molekularem Sauerstoff als Oxidationsmittel und
e) einem mindestens ein Edelmetall und/oder eine Edelmetallverbindung der achten Nebengruppe des Periodensystems der Elemente enthaltenden Katalysatorsystem,

dadurch gekennzeichnet, dass man die Umsetzung in Gegenwart von Katalysatorsystemen e) durchführt, die als weitere Komponente mindestens eine oxidierend wirkende chinoide Verbindung und/oder mindestens eine Verbindung enthalten, welche unter den Reaktionsbedingungen in eine oxidierend wirkende chinoide Verbindung überführt werden kann.

Die Mitverwendung von Chinonen bei der phosgenfreien Herstellung von Urethanen ist zwar aus DE-OS 2 808 980 und DE-OS 2 838 754 bereits bekannt, jedoch kann die Lehre dieser Vorveröffentlichungen in keinen unmittelbaren Zusammenhang mit dem erfindungsgemässen Verfahren gebracht werden. So erwähnt beispielsweise die DE-OS 2 808 980 die Möglichkeit der Verwendung von Chinonen als oxidierend wirkende Verbindungen bei der Herstellung von Urethanen durch Umsetzung von aromatischen Nitroverbindungen mit Alkoholen und Kohlenmonoxid in Gegenwart von Selen und/oder Selenverbindungen, sowie aromatische Aminoverbindungen und/oder aromatische Harnstoffverbindungen aufweisenden Katalysatorsystemen. Irgendwelche Hinweise auf die cokatalytische Wirkung von Chinonen bei der Oxicarbonylierung von primären Aminen unter Verwendung der erfindungsgemässen Katalysatorsysteme sind dieser Vorveröffentlichung nicht zu entnehmen. Die DE-OS 2 838 754 befasst sich ebenfalls mit der Herstellung von Urethanen durch Umsetzung von aromatischen Nitroverbindungen mit Alkoholen und Kohlenmonoxid in Gegenwart von Schwefel und/oder Selen und/oder Verbindungen dieser Elemente enthaltenden Katalysatorsystemen, wobei als, allerdings weniger bevorzugte, Cokatalysatoren auch Chinone erwähnt sind. Auch hier kann jedoch gesagt werden, dass sowohl die Ausgangsmaterialien als auch die zum Einsatz gelangenden Katalysatorsysteme von den erfindungsgemäss einzusetzenden Ausgangsmaterialien und den erfindungsgemässen Katalysatorsystemen so verschieden sind, dass die Lehre der DE-OS 2 838 754 keinerlei Rückschlüsse auf das erfindungsgemässe Verfahren gestattet.

Ausgangsmaterialien für das erfindungsgemässe Verfahren sind

a) primäre Amine,
b) hydroxylgruppenaufweisende organische Verbindungen,
c) Kohlenmonoxid und
d) molekularer Sauerstoff als Oxidationsmittel.

Geeignete primäre Amine a) sind beliebige organische Verbindungen mit mindestens einer primären Aminogruppe, insbesondere beliebige primäre Amine mit mindestens einer aliphatisch, cycloaliphatisch, aromatisch oder heterocyclisch gebundenen Aminogruppe, die gegebenenfals noch weitere funktionelle Gruppen enthalten können. Vorzugsweise

werden aromatische oder aliphatische Mono- oder Diamine, insbesondere Monoamine eingesetzt, die, von den primären Aminogruppen abgesehen, keine weiteren oxidierbaren Substituenten aufweisen. Die Amine weisen im allgemeinen ein Molekulargewicht von 31 bis 3000, vorzugsweise 31 bis 400 und insbesondere 31 bis 200 auf.

Beispiele geeigneter aromatischer bzw. heterocyclischer Amine sind: Anilin, 1,2-Diaminobenzol, 1,4-Diaminobenzol, die isomeren Chloraniline, 3,4-Dichloranilin, 4-Isopropylanilin, p-Toluidin, Chlortoluidine, Xylidine, Alkoxyaniline, 4-Pentachlorethylanilin, 2-Nitroanilin, 3-Nitroanilin, 4-Nitroanilin, 2,3-Diamino-toluol, 2,4-Diamino-toluol, 2,6-Diamino-toluol, 2,5-Diamino-toluol, 3,4-Diamino-toluol, 3,5-Diamino-toluol, 2-Amino-4-nitrotoluol, 2-Amino-3-nitritoluol, 2-Amino-5-nitrotoluol, Aminophenole, Diaminoxylole, Aminonitroxylole, Aminonaphthaline, Aminoanthracene, Chloraminobenzoesäuren, Chloraminobenzoesäureester, Aminobenzolsulfonsäuren, 4,4'-Diaminodiphenylmethan, 2,2'-Diaminodiphenylmethan, 2,4-Diaminodiphenylmethan, Tris-(4-aminophenyl)-methan, Aminopyridine, Aminochinoline, Aminopyrrole, Aminofurane, Aminothiophene oder 2-Aminobenzothiazol.

Beispiele geeigneter cycloaliphatischer primärer Amine sind: Aminocyclobutan, Aminocyclopentan, Cyclohexylamin, 1,2-Diaminocyclohexan, 1,3-Diaminocyclohexan, 1,4-Diaminocyclohexan, Bis-(aminocyclohexyl)-methane, Tris-(aminocyclohexyl)-methane.

Beispielhaft für die Gruppe der aliphatischen primären Amine seien genannt: Methylamin, Ethylamin, 1-Propylamin, 2-Propylamin, 1-Butylamin, 2-Butylamin, Isobutylamin, tert.-Butylamin, 1-Pentylamin, 1-Hexylamin, 1-Heptylamin, 1-Octylamin, 1-Decylamin, 1-Dodecylamin, Ethylendiamin, 1,2-Diaminopropan, 1,3-Diaminopropan, Diaminobutane, Diaminopentane, Diaminohexane, Diaminooctane, Diaminodecane, Benzylamin, Bis-(aminomethyl)-cyclohexane, Bis-(aminomethyl)-benzol, ω-Aminocarbonsäureester, ω-Aminocarbonsäurenitrile.

Besonders bevorzugte primäre Amine sind aromatische primäre Amine wie insbesondere Anilin, substituierte Aniline, die isomeren Diaminotoluole und 4,4'-Diaminodiphenylmethan.

Geeignete hydroxylgruppenaufweisende organische Verbindungen b) sind beliebige, mindestens eine alkoholisch oder phenolisch gebundene Hydroxylgruppe aufweisende organische Verbindungen, die im allgemeinen ein Molekulargewicht von 32 bis 2000, vorzugsweise 32 bis 300 aufweisen. Die Alkohole sind gegenüber den Phenolen bevorzugt.

Geeignete Alkohole sind beispielsweise solche des Molekulargewichtsbereichs 32-300, wobei es sich bei diesen Alkoholen um beliebige lineare oder verzweigte ein- oder mehrwertige Alkanole oder Alkenole, um beliebige ein- oder mehrwertige Cycloalkanole, Cycloalkenole oder Aralkanole handeln kann. Auch beliebige inerte Substituenten aufweisende Alkohole sind geeignet. Geeignete Substituenten sind z.B. Halogenatome, Sulfoxidgruppen, Sulfongruppen, Carbonyl- oder Carbonsäureestergruppen. Auch Etherbrücken aufweisende Alkohole sind grundsätzlich geeignet. Beispiele geeigneter Alkohole sind: Methanol, Ethanol, n-Propanol, Isopropanol, n-Butanol, n-Pentanol, n-Hexanol, Cyclohexanol, Benzylalkohol, Chlorethanol, Ethylenglykol, Diethylenglykol, Propylenglykol, Dipropylenglykol, Glycerin, Hexantriol oder Trimethylolpropan. Falls höherwertige Alkohole verwendet werden, sollten beim erfindungsgemässen Verfahren einbasische Amine als Ausgangskomponente a) eingesetzt werden. Umgekehrt sollten bei der Verwendung von höherfunktionellen Aminen a) einwertige Hydroxylverbindungen b) verwendet werden. Bevorzugt werden beim erfindungsgemässen Verfahren einwertige aliphatische Alkohole mit 1 bis 6 Kohlenstoffatomen eingesetzt.

Geeignete Phenole sind insbesondere solche im Molekulargewichtsbereich von 94-600, insbesondere 94-300, wie beispielsweise Phenol, α-Naphthol, β-Naphthol, Anthranol, Phenanthrol, Hydroxybenzofurane, Hydroxychinoline sowie mehrwertige Phenole wie z.B. Dihydroxybenzole, Dihydroxynaphthaline, 4,4'-Dihydroxydiphenylmethan, Bisphenol A, Pyragallol oder Phloroglucin. Auch beliebige inerte Substituenten aufweisende Phenole der obengenannten Typen sind geeignet. Geeignete Substituenten sind z.B. Halogenatome, Sulfoxidgruppen, Sulfongruppen, Carbonyl- oder Carbonsäureestergruppen, Nitrogruppen, Alkylgruppen, Arylgruppen, Alkoxygruppen und Aroxygruppen.

Besonders bevorzugte Phenole sind Phenol, die isomeren Chlorphenole, Bisphenol A, 2-Isopropoxyphenol und 7-Hydroxy-2,2-dimethyl-2,3-dihydrobenzofuran.

Bei der Durchführung des erfindungsgemässen Verfahrens werden die organische Hydroxylgruppen enthaltenden Verbindungen im allgemeinen in solchen Mengen eingesetzt, dass für jedes Mol an im Reaktionsgemisch vorliegenden primären Aminogruppen 1 bis 200, vorzugsweise 1 bis 50 Mol an Hydroxylgruppen vorliegen. Da im allgemeinen relativ billige und unter den Reaktionsbedingungen flüssige Hydroxylverbindungen zum Einsatz gelangen, dient deren Überschuss als Reaktionsmedium (Lösungsmittel) für das erfindungsgemässe Verfahren.

Als weiterer Reaktionspartner c) kommt beim erfindungsgemässen Verfahren Kohlenmonoxid zum Einsatz. Dieses Ausgangsmaterial wird im allgemeinen in einer Menge eingesetzt, die 1 bis 30 Mol Kohlenmonoxid pro Mol zu erzeugendem Urethan entspricht, d.h. es kommen für jedes Mol an im Reaktionsgemisch vorliegenden primären Aminogruppen im allgemeinen 1 bis 30 Mol Kohlenmonoxid zum Einsatz.

Als Oxidationsmittel d) kann molekularer Sauerstoff in reiner Form oder in Form von Gemischen mit Inertgas, wie Stickstoff oder Kohlendioxid, insbesondere in Form von Luft, eingesetzt werden. In Gegenwart von molekularem Sauerstoff erfolgt die Oxycarbonylierung nach folgender allgemeiner Reaktionsgleichung:

$$R\ NH_2 + \frac{1}{2} O_2 + CO + R'OH \rightarrow R\ NHCO_2R' + H_2O$$

d.h., je gebildeter Urethangruppe wird 1 Mol Kohlenmonoxid und $^1/_2$ Mol Sauerstoff benötigt. Im allgemeinen wird molekularer Sauerstoff bezüglich der

umzusetzenden Aminogruppen in etwa stöchiometrischer bis etwa fünffacher Überschuss-Menge eingesetzt. Bei Verwendung von gegenüber Oxidationsmitteln empfindlichen Alkoholen c) kann es oft auch zweckmässig sein, den als Oxidationsmittel d) verwendeten Sauerstoff im Unterschuss, bezogen auf die Aminogruppe der Komponente a), einzusetzen. Dies bedeutet, dass es durchaus zweckmässig sein kann, den Sauerstoff lediglich in einer Menge einzusetzen, die 60 bis 100% der gemäss obiger Gleichung erforderlichen, äquivalenten Menge entspricht, da bei Verwendung von oxidationsempfindlichen Alkoholen die durch unerwünschte Oxidationsreaktionen hervorgerufenen Ausbeuteverluste schwerer ins Gewicht fallen können als die durch Verwendung unterschüssiger Mengen Oxidationsmittel d) hervorgerufenen Ausbeuteverluste. Bei Verwendung von unterschüssigen Mengen an Sauerstoff, entstehen im übrigen Reaktionsgemische, in denen leicht wiedergewinnbares Ausgangsamin a) vorliegt, welches erneut für das erfindungsgemässe Verfahren eingesetzt werden kann. Durch unerwünschte Oxidationsreaktionen vernichteter Alkohol kann jedoch nicht wiedergewonnen werden.

Das erfindungsgemässe Verfahren wird in Gegenwart von Katalysatorsystemen e) durchgeführt. Diese Katalysatorensysteme enthalten e1) mindestens ein Edelmetall und/oder mindestens eine Edelmetallverbindung der achten Nebengruppe des Periodensystems der Elemente und e2) mindestens eine oxidierend wirkende chinoide Verbindung und/oder mindestens eine Verbindung, welche unter den Reaktionsbedingungen in eine oxidierend wirkende chinoide Verbindung überführt werden kann.

Bei der Katalysatorkomponente e1) handelt es sich entweder um freie Edelmetalle der achten Nebengruppe des Periodensystems oder um Verbindungen dieser Metalle. Besonders vorteilhaft werden die Edelmetalle als im Reaktionsgemisch lösliche Verbindungen zugegeben, beispielsweise Chloride, Bromide, Jodide, Chlorokomplexe, Bromokomplexe, Jodokomplexe, Acetate, Acetylacetonate u.a. lösliche Edelmetallverbindungen. Bevorzugte Edelmetalle sind Palladium, Ruthenium und Rhodium. Besonders bevorzugt wird Palladium, insbesondere in Form von löslichem Palladiumchlorid oder Palladiumacetat eingesetzt.

Bevorzugte Konzentrationen für die Katalysatorkomponente e1) liegen im allgemeinen bei 3 bis 1000 ppm, vorzugsweise bei 5 bis 100 ppm, berechnet als Edelmetall und auf das gesamte Reaktionsgemisch bezogen, einschliesslich des eventuell mitverwendeten Lösungsmittels. Höhere Edelmetall-Konzentrationen sind zwar möglich, wegen der möglichen Edelmetallverluste jedoch unwirtschaftlich, zumal eine weitere Steigerung der Urethanausbeuten nicht mehr erfolgt.

Bei der Katalysatorkomponente e2) handelt es sich um eine oxidierend wirkende chinoide Verbindung und/oder um eine Verbindung, welche unter den Reaktionsbedingungen in eine oxidierend wirkende chinoide Verbindung überführt werden kann. Unter chinoiden Verbindungen sind Verbindungen zu verstehen, wie sie z.B. in «The Chemistry of the Quinoid Compounds» Part I and I' (London, Wiley 1974, Herausgeber: Patai) beschrieben werden und wie sie z.B. als Farbstoffe oder Farbstoffvorprodukte vielfach hergestellt werden. Grundsätzlich sind alle derartigen chinoiden Verbindungen als erfindungsgemässe Katalysatorkomponente e2) geeignet, welche unter den erfindungsgemässen Reaktionsbedingungen dazu befähigt sind, das in der Katalysatorkomponente e1) vorliegende Edelmetall von der Oxidationsstufe Null in eine positive Oxidationsstufe aufzuoxidieren, insbesondere solche, die unter den erfindungsgemässen Bedingungen dazu befähigt sind, das besonders bevorzugt eingesetzte Palladium von der Oxidationsstufe Null in die Oxidationsstufe +2 zu überführen.

Anstelle derartiger chinoider Verbindungen können jedoch als erfindungsgemässe Katalysatorkomponente e2) auch in chinoide Verbindungen der genannten Art überführbare Verbindungen eingesetzt werden, d.h. Verbindungen, die durch eine Oxidationsreaktion [hervorgerufen durch die beim erfindungsgemässen Verfahren mitzuverwendenden Oxidationsmittel d)], Solvolyse oder Eliminierungsreaktion in eine derartige chinoide Verbindung übergehen.

Geeignete chinoide Katalysatorkomponenten e2) sind ortho- und para-Chinone, mehrkernige Chinone und heterocyclische Chinone in substituierter und nicht-substituierter Form sowie deren Imino-, N-Alkyl- oder N-Aryliminoderivate wie z.B. o-Tetrachlorbenzochinon, p-Tetrachlorbenzochinon, 2,5-Dichlor-3,6-dihydroxy-p-benzochinon, 2-Chlorphenyl-1,4-benzochinon, 2,3-Dichlornaphthochinon, Anthrachinon, 1-Chloranthrachinon, 7-Chlor-4-hydroxy-1,10-anthrachinon, 1-Nitroanthrachinon-2-carbonsäure, 1,5-Dichloranthrachinon, 1,8-Dichloranthrachinon, 2,6-Dichloranthrachinon, 1,4-Dihydroxyanthrachinon, Acenaphthylendion, 5,7-Dichlor-1H-indol-2,3-dion, Indigo oder 1,4-Dihydro-2,3-chinoxalindion.

Auch polymere chinoide Verbindungen, wie sie z.B. von H.G. Cassidy und K.A. Kun in «Oxidation-Reduction Polymers» (Polymer Reviews Vol 11, Interscience Publ. New York 1965) beschrieben worden sind, sind als Katalysatorkomponente e2) geeignet.

Bevorzugte chinoide Verbindungen sind zur Erhöhung der Oxidationsfähgkeit mit einer oder mehreren elektronenziehenden Substituenten wie z.B. Chlor, Brom, Cyan-, Nitro-, Carbonsäure- oder Sulfonsäuregruppen substituiert. Als Katalysatorkomponente e2) besonders bevorzugte chinoide Verbindungen sind z.B. o-Tetrachlorbenzochinon, p-Tetrachlorbenzochinon, 2,5-Dichlor-3,6-dihydroxy-p-benzochinon, 2,3-Dichlor-naphthochinon, 7-Chlor-4-hydroxy-1,10-anthrachinon, 1,5-Dichloranthrachinon und 1,8-Dichloranthrachinon.

Als Katalysatorkomponente e2) geeignete Vorstufen chinoider Verbindungen sind z.B. Ketale der entsprechenden chinoiden Katalysatorkomponenten sowie hydrierte Formen jener Komponenten, insbesondere die entsprechenden Hydrochinone. Ebenso werden aromatische Amine und mehrkernige Aromaten, die z.B. durch Sulfonsäure-, Carbonsäure-, Nitro- oder Cyangruppen substituiert sind oder die bereits eine Oxogruppe im Ringsystem tragen, unter den oxidativen Reaktionsbedingungen, z.B. durch

molekularen Sauerstoff, in erfindungsgemässe chinoide Katalysatorkomponenten e2) überführt. Geeignete Vorstufen für die chinoide Katalysatorkomponente e2) sind z.B. die Hydrochinone und Ketale der obengenannten Chinone der 5-Amino-2-(phenylamino)benzolsulfonsäure, 5-Amino-2-[(4-chlorphenyl)amino]-benzolsulfonsäure, 4,4'-Diamino-(1,1'-biphenyl)-3,3'-disulfonsäure, 2-Aminobenzolsulfonsäure sowie Benzanthron-3-carbonitril.

Die erfindungsgemässe Katalysatorkomponente e2) wird dem Reaktionssystem im allgemeinen in Konzentrationen von 0,1 Gew.-% bis 5 Gew.-%, bevorzugt in Konzentrationen von 0,5 bis 3 Gew.-%, bezogen auf die Gesamtmenge des Reaktionsgemisches inklusive etwa mitverwendeter Lösungsmittel, beigefügt.

Die erfindungsgemäss einzusetzende Katalysatorkomponente e) kann gegebenenfalls als weitere Komponenten bestimmte Metallverbindungen e3) und/oder tert.-Amine e4) enthalten.

Bei der gegebenenfalls mitzuverwendenden Katalysatorkomponente e3) handelt es sich um Magnesiumverbindungen, insbesondere anorganische oder organische Salze des Magnesiums oder um unter den Reaktionsbedingungen zu Redoxreaktionen befähigte Verbindungen von Elementen der dritten bis fünften Hauptgruppe und/oder der ersten bis achten Nebengruppe des Periodensystems der Elemente. Vorzugsweise werden als gegebenenfalls mitzuverwendende Katalysatorkomponente e3) im Reaktionsgemisch zumindest teilweise lösliche Verbindungen der Metalle mit den Ordnungszahlen 12, 22 bis 29, 41, 47, 58 und 92 eingesetzt. Besonders bevorzugte Katalysatorkomponenten e3) sind die Acetate, Nitrate oder Chloride des Chroms, Mangangs, Kobalts, Kupfers, Cer oder des Magnesiums, gegebenenfalls in Form der Hydrate oder Aminkomplexe dieser Metallsalze. In Verbindung mit aktivierend wirkenden Chloriden, wie z.B. Ammoniumchloriden, können auch die Oxide der beispielhaft genannten Metalle als Katalysatorkomponente e3) verwendet werden. Falls an eine Mitverwendung der Katalysatorkomponente e3) gedacht ist, wird diese im allgemeinen in einer ein- bis zehnfach molaren Menge, bezogen auf die Katalysatorkomponente e1) eingesetzt. Dies bedeutet im allgemeinen, dass die Katalysatorkomponente e3) in Mengen von bis zu 0,1 Gew.-%, bezogen auf das Gesamtgewicht des Reaktionsgemisches, inklusive gegebenenfalls mitverwendetem Lösungsmittel, mitverwendet werden kann.

Bei der gegebenenfalls mitzuverwendenden Katalysatorkomponente e4) handelt es sich um beliebige tertiäre Amine, welche im Katalysatorsystem die Funktion eines Komplexbildners für die oxidierte Form der Katalysatorkomponente e1) und gegebenenfalls für die Katalysatorkomponente e3) erfüllt, falls die komplexierende Wirkung der im Reaktionsgemisch vorliegenden Ausgangsverbindungen zu diesen Zwecken nicht ausreicht. Prinzipiell sind alle beliebigen tertiären Amine geeignet, d.h. solche, die aliphatisch, cycloaliphatisch, araliphatisch und/oder aromatisch gebundene tertiäre Aminogruppen oder Teil eines heterocyclischen Rings bildende tertiäre Aminogruppen enthalten. Geeignete tertiäre Amine sind beispielsweise Triethylamin, Diisopropylmethylamin, Cyclohexyldiethylamin, Triphenylamin, N,N-Diethylanilin, N-Phenylpiperidin, Pyridin, Chinolin, 1,4-Diaza(2.2.2)-bicyclooctan oder Pyrimidin. Bevorzugte tertiäre Amine e4) sind Triethylamin, N,N-Diethylanilin und Pyridin. Die genannten tertiären Amine können auch als Metallsalzkomplexe der Katalysatorkomponente e1) und gegebenenfalls e3) eingesetzt werden. Setzt man die Katalysatorkomponente e1) und/oder gegebenenfalls e3) in oxidischer Formein, so ist es vorteilhaft zu deren Aktivierung die tertiären Amine in Form von Hydrochloriden einzusetzen. Die gegebenenfalls zu verwendende Katalysatorkomponente e4) wird in Mengen von bis zu 10 Gew.-%, vorzugsweise in 0,5 bis 6 Gew.-%, bezogen auf den Gesamteinsatz der Reaktion, einschliesslich des eventuell verwendeten Lösungsmittels, eingesetzt. Es ist jedoch auch möglich, die Katalysatorkomponente e4) in höheren Mengen einzusetzen.

Die erfindungsgemässe Umsetzung kann in Gegenwart oder in Abwesenheit eines Lösungsmittels durchgeführt werden. Im allgemeinen dient die vorzugsweise im Überschuss eingesetzte organische Hydroxylverbindung b) als Lösungsmittel. Die Mitverwendung inerter Lösungsmittel, die bis zu 80 Gew.-% des gesamten Reaktionsansatzes ausmachen können, ist jedoch auch möglich. Die Menge des Lösungsmittels muss, gleichgültig, ob es sich um im Überschuss eingesetzte Hydroxylverbindung oder um inertes Lösungsmittel handelt, so bemessensein, dass die Reaktionswärme der exothermen Urethanbildung ohne unzulässige Temperaturerhöhung abgeführt werden kann. Im allgemeinen wird daher das erfindungsgemässe Verfahren unter Verwendung einer Konzentration an Aminoverbindungen a) von 5 bis 50 Gew.-%, vorzugsweise 5 bis 20 Gew.-%, bezogen auf das Gesamtreaktionsgemisch inklusive dem Lösungsmittel, durchgeführt.

Brauchbare Lösungsmittel sind gegenüber den Reaktionskomponenten und dem Katalysatorsystem inerte Lösungsmittel, wie beispielsweise aromatische, cycloaliphatische und aliphatische Kohlenwasserstoffe, die gegebenenfalls Halogen-substituiert sein können, wie Benzol, Toluol, Xylol, Chlorbenzol, Dichlorbenzol, Trichlorbenzol, Chlornaphthalin, Cyclohexan, Methylcyclohexan, Chlorcyclohexan, Methylenchlorid, Tetrachlorkohlenstoff, Tetrachlorethan, Trichlortrifluorethan u.ä. Verbindungen sowie tertiäre Amine wie sie als Katalysatorkomponente e4) beschrieben wurden.

Die Reaktionstemperatur liegt im allgemeinen zwischen 100°C und etwa 300°C, insbesondere zwischen 100°C und 250°C und besonders vorteilhaft im Bereich von 140°C bis 220°C. Der Druck muss so bemessen sein, dass stets das Vorliegen einer flüssigen Phase gewährleistet ist und liegt im allgemeinen im Bereich von 5 bis 500 bar, besonders vorteilhaft im Bereich von 30 bis 300 bar. Je nach eingesetztem primären Amin bzw. Hydroxylverbindung beträgt die für den quantitativen Umsatz benötigte Reaktionszeit zwischen wenigen Minuten und mehreren Stunden.

Das erfindungsgemässe Verfahren kann diskontinuierlich oder kontinuierlich durchgeführt werden. Es ist vorteilhaft, ein Lösungsmittel zu wählen, in

welchem das Verfahrensprodukt (Urethan) gut löslich ist. Nach dem Entspannen des Reaktionsmediums und Abkühlen auf 50°C bis 80°C fallen die Katalysatorkomponenten e1) bis e3) sowie in komplexierter Form gegebenenfalls e4) in vielen Lösungsmitteln weitgehend bis vollständig aus; in manchen Fällen ist es vorteilhaft, das Reaktionsgemisch auf 70 bis 50% des ursprünglichen Volumens einzuengen, um die Katalysatorfällung zu erreichen. Das Katalysatorgemisch kann durch Filtrieren oder Zentrifugieren von der urethanhaltigen Lösung abgetrennt werden. Die in dieser Weise abgetrennten Katalysatorkomponenten e1) bis e3) und gegebenenfalls e4) sind in den meisten Fällen rückführbar und wiederum katalytisch aktiv, obwohl sie in chemisch veränderter Form vorliegen. Das Urethan kann aus dem Filtrat z.B. durch Verdampfen des Lösungsmittels ausgeschieden und z.B. durch Vakuumdestillation oder Kristallisation gereinigt werden. In analoger Weise kann die Aufarbeitung bei Verwendung von salzartigen anorganischen Oxidationsmitteln oder chinonartigen Oxidationsmitteln erfolgen. Das nach der Reaktion in reduzierter Form erhaltene Oxidationsmittel enthält dann weitgehend die Katalysatorkomponenten e1) bis e3) und kann z.B. in deren Anwesenheit reoxidiert und gemeinsam mit diesen Katalysatorkomponenten in die Reaktion rückgeführt werden.

Die erfindungsgemässen Verfahrensprodukte (Urethane) eignen sich als Schädlingsbekämpfungsmittel bzw. als Zwischenprodukte zur Herstellung von Schädlingsbekämpfungsmitteln. Sie sind jedoch in erster Linie als Ausgangsmaterialien zur Herstellung der ihnen zugrundeliegenden Isocyanate von Interesse. Diese Herstellung der Isocyanate erfolgt durch an sich bekannte thermische Spaltung der erfindungsgemässen Verfahrensprodukte.

Das Verfahren wird durch die folgenden Beispiele illustriert, ohne damit die Erfindung auf die in den Beispielen gegebenen Bedingungen einzuschränken. Die Urethanausbeuten beziehen sich jeweils auf eingesetztes Amin a) in Mol-%.

*Beispiel 1* [Vergleichsbeispiel ohne Katalysatorkomponente e2)]

In einem emaillierten 1,3-l-Edelstahlautoklaven werden 474 g eines Gemisches folgender Zusammensetzung vorgelegt: 42 ppm Palladiumacetat, 211 ppm Kupfer-II-acetatmonohydrat, 91,4 Gew.-% Ethanol und 8,6 Gew.-% Anilin. Bei Raumtemperatur werden sodann 100 bar Kohlenmonoxid und 25 bar Luft aufgepresst. Unter Rühren wird auf 180°C aufgeheizt und eine Stunde bei dieser Temperatur reagieren gelassen. Nach Abkühlen auf Raumtemperatur wird entspannt und eine zweite gleichartige Reaktionsphase mit frischem CO/Luftgemisch durchgeführt. Es werden somit insgesamt ca. 1,4 Oxidationsäquivalente bezüglich Anilin in Form von Luftsauerstoff eingebracht. Aus der gaschromatographischen Analyse des flüssigen Reaktionsgemisches errechnet sich die Ausbeute an Phenylurethan zu 4,5% bezogen auf eingesetztes Anilin.

*Beispiele 2 bis 9*

Diese Beispiele demonstrieren die katalytische Wirksamkeit der Katalysatorkomponente e2) im Vergleich zu Beispiel 1. In den Beispielen 2 bis 6 ist die Katalysatorkomponente e2) eine chinoide Verbindung, in den Beispielen 7 bis 9 ist sie Vorstufe einer chinoiden Verbindung. Es wird wie in Beispiel 1 gearbeitet; eingesetzt werden 483 g eines Gemisches folgender Zusammensetzung: 41 ppm Palladiumacetat, 207 ppm Kupfer-II-acetatmonohydrat, 1,8 Gew.-% Katalysatorkomponente e2), 89,8 Gew.-% Ethanol und 8,4 Gew.-% Anilin. Ergebnisse siehe Tabelle 1.

Tabelle 1

| Beispiel Nr. | Katalysatorkomponente e2) | Phenylurethan Ausbeute % |
|---|---|---|
| 2 | ortho-Tetrachlorbenzochinon | 46,0 |
| 3 | para-Tetrachlorbenzochinon | 64,0 |
| 4 | 2,5-Dichlor-3,6-dihydroy-para-benzochinon | 47,5 |
| 5 | 2,3-Dichlornaphthochinon | 52,5 |
| 6 | 1,5-Dichloranthrachinon | 40,9 |
| 7 | Benzanthron-3-carbonitril | 27,1 |
| 8 | 5-Amino-2-(phenylamino)-benzolsulfonsäure | 29,0 |
| 9 | 4,4'-Diamino-(1,1'-biphenyl)-3,3'-disulfonsäure | 32,0 |

*Beispiel 10*

Es wird wie in Beispiel 1 gearbeitet; es werden 487 g eines Gemisches mit folgender Zusammensetzung eingesetzt: 41 ppm Palladiumacetat, 206 ppm Kupfer-II-acetatmonohydrat, 1,7 Gew.-% p-Tetrachlorbenzochinon, 0,8 Gew.-% N,N-Diethylanilin, 89,0 Gew.-% Ethanol und 8,4 Gew.-% Anilin. Ausbeute Phenylurethan: 72,6%.

*Beispiel 11*

Es wird wie in Beispiel 1 gearbeitet; es werden 493 g eines Gemisches mit folgender Zusammensetzung eingesetzt: 41 ppm Palladiumacetat, 207 ppm Kupfer-II-acetatmonohydrat, 1,8 Gew.-% p-Tetrachlorbenzochinon, 48,3 Gew.-% Ethanol, 8,4 Gew.-% Anilin und 41,5 Gew.-% ortho-Dichlorbenzol. Ausbeute Phenylurethan: 71,8%.

*Beispiele 12 bis 17*

Diese Beispiele zeigen die katalytische Wirksamkeit verschiedener Edelmetalle als Katalysatorkomponente e1): Es wird wie in Beispiel 1 gearbeitet, jedoch mit 483 g eines Einsatzgemisches mit folgender Zusammensetzung: 104 ppm Katalysatorkomponente e1), 207 ppm Kupfer-II-acetatmonohydrat, 1,8 Gew.-% p-Tetrachlorbenzochinon, 89,8 Gew.-% Ethanol und 8,4 Gew.-% Anilin. Ergebnisse siehe Tabelle 2. Beispiel 12 ist ein Vergleichsbeispiel ohne die erfindungsgemässe Katalysatorkomponente e1).

Tabelle 2

| Beispiel Nr. | Katalysatorkomponente e1) | Phenylurethan Ausbeute % |
|---|---|---|
| 12 | — | 1,0 |
| 13 | $RuCl_3$ | 55,6 |
| 14 | $RhCl_3$ | 38,9 |
| 15 | $PdCl_2$ | 57,2 |
| 16 | $IrCl_3$ | 5,5 |
| 17 | $PtCl_2$ | 4,4 |

*Beispiel 18*

Dieses Beispiel belegt, dass die Katalysatorkomponenten e1) und e2) auch in Abwesenheit der Katalysatorkomponenten e3) und e4) die Urethanbildung katalysieren: Es wird wie in Beispiel 1 gearbeitet, jedoch werden 482 g eines Einsatzgemisches mit folgender Zusammensetzung vorgelegt: 44 ppm Palladiumchlorid, 1,8 Gew.-% p-Tetrachlorbenzochinon, 89,8 Gew.-% Ethanol und 8,4 Gew.-% Anilin. Ausbeute Phenylurethan: 54,6%.

*Beispiele 19 bis 23*

Es wird wie in Beispiel 1 gearbeitet, jedoch wird ein 0,7-l-Edelstahlautoklav mit 223 g Einsatzgemisch folgender Zusammensetzung verwendet: 22 ppm Palladiumchlorid, 1,8 Gew.-% p-Tetrachlorbenzochinon, 90 ppm Katalysatorkomponente e3), 89,8 Gew.-% Ethanol und 8,4 Gew.-% Anilin. Ergebnisse siehe Tabelle 3.

Tabelle 3

| Beispiel Nr. | Katalysatorkomponente e3) | Polyurethan Ausbeute % |
|---|---|---|
| 19 | $Cr(NO_3)_3 \cdot 9\ H_2O$ | 59,4 |
| 20 | $Mn(OAc)_2 \cdot H_2O$ | 60,2 |
| 21 | $Co(OAc)_2 \cdot H_2O$ | 65,3 |
| 22 | $Cu(OAC)_2 \cdot H_2O$ | 62,1 |
| 23 | $Mg(NO_3)_2 \cdot 6\ H_2O$ | 60,7 |

*Beispiel 24*

Dieses Beispiel zeigt die katalytische Wirksamkeit eines rückgeführten Katalysators: Aus einem Produktgemisch, das wie in Beispiel 3 angegeben erhalten wird, wird der ausgefallene Feststoff abfiltriert und bei 50°C getrocknet. In einem 0,3-l-Edelstahlautoklaven werden unter den Bedingungen von Beispiel 3 111 g eines Gesamteinsatzes mit folgender Zusammensetzung zur Reaktion gebracht: 1,8 Gew.-% rückgewonnenes Katalysatorgemisch, 8,4 Gew.-% Anilin und 89,8 Gew.-% Ethanol. Ausbeute Phenylurethan: 62,4%.

*Beispiele 25 bis 29*

In einem 0,3-l-Edelstahlautoklaven werden 111,4 g eines Gemisches folgender Zusammensetzung vorgelegt: 90 ppm Palladiumacetat, 450 ppm Kupferacetatmonohydrat, 1,8 Gew.-% Tetrachlor-p--benzochinon, 8,4 Gew.-% Anilin und 89,8 Gew.-% Hydroxykomponente c) (siehe Tebelle 4). Bei Raumtemperatur werden 100 bar Kohlenmonoxid und 25 bar Luft aufgepresst; es werden somit ca. 0,7 Oxidationsäquivalente bezüglich Anilin in Form von Luftsauerstoff eingebracht. Unter Rühren lässt man eine Stunde bei 180°C reagieren. Nach dem Abkühlen werden durch gaschromatographische Bestimmung nachstehende Urethanausbeuten erhalten.

Tabelle 4

| Hydroxykomponente c) | Ausbeute N-Phenylurethan % |
|---|---|
| Ethanol | 58 |
| 1-Propanol | 64 |
| 2-Propanol | 62 |
| Cyclohexanol | 34 |
| Benzylalkohol | 20 |

*Beispiel 30*

In einem 0,7-l-Edelstahlautoklaven werden 216,7 g eines Gemisches folgender Zusammensetzung vorgelegt: 50 ppm Palladiumacetat, 250 ppm Kupferacetatmonohydrat, 2,2 Gew.-% 2,3-Dichlornaphthochinon, 5,5 Gew.-% tert.-Butylamin und 92,3 Gew.-% Ethanol. Bei Raumtemperatur werden 100 bar CO und 25 bar Luft aufgepresst; es werdensomit ca. 1,2 Oxidationsäquivalente bezüglich tert.-Butylamin in Form von Luftsauerstoff eingebracht. Unter Rühren lässt man eine Stunde bei 180°C reagieren. Nach dem Abkühlen wird durch gaschromatographische Bestimmung N-tert.-O-ethylurethan mit 32% Ausbeute nachgewiesen.

**Patentansprüche**

1. Verfahren zur Herstellung von Urethanen durch Umsetzung von
a) primären Aminen mit
b) mindestens eine Hydroxylgruppe enthaltenden organischen Verbindungen und
c) Kohlenmonoxid in Gegenwart von
d) molekularem Sauerstoff als Oxidationsmittel und
e) einem mindestens ein Edelmetall und/oder eine Edelmetallverbindung der achten Nebengruppe des Periodensystems der Elemente enthaltenden Katalysatorsystem,

dadurch gekennzeichnet, dass man die Umsetzung in Gegenwart von Katalysatorsystemen e) durchführt, die als weitere Komponente mindestens eine oxidierend wirkende chinoide Verbindung und/oder mindestens eine Verbindung enthalten, welche unter den Reaktionsbedingungen in eine oxidierend wirkende chinoide Verbindung überführt werden kann.

2. Verfahren gemäss Anspruch 1, dadurch gekennzeichnet, dass man als Katalysatorsystem e) ein solches verwendet, welches als weitere Komponente mindestens eine unter den Reaktionsbedingungen zu Redoxreaktionen befähigte Verbindung von Elementen der dritten bis fünften Hauptgruppe und/oder der ersten bis achten Nebengruppe des Periodensystems der Elemente enthält.

3. Verfahren gemäss Anspruch 1 bis 2, dadurch gekennzeichnet, dass man als Katalysatorsystem e) ein solches verwendet, welches mindestens ein tertiäres Amin als weitere Komponente enthält.

4. Verfahren gemäss Anspruch 1 bis 3, dadurch gekennzeichnet, dass man als Katalysatorsystem e) ein solches verwendet, welches, bezogen auf das Gesamtgewicht des Reaktionsansatzes, 5 ppm bis 100 ppm, berechnet als Edelmetall, mindestens eines Edelmetalls und/oder mindestend einer Edelmetallverbindung enthält.

5. Verfahren gemäss Anspruch 1 bis 4, dadurch gekennzeichnet, dass man als Katalysatorsystem e) ein solches verwendet, welches, bezogen auf das Gesamtgewicht des Reaktionsgemisches, 0,1 bis 5 Gew.-% mindestens einer oxidierend wirkenden chinoiden Verbindung und/oder einer Verbindung enthält, welche unter den Reaktionsbedingungen in eine oxidierend wirkende chinoide Verbindung überführt werden kann.

6. Verfahren gemäss Anspruch 1 bis 5, dadurch gekennzeichnet, dass man als Katalysatorsystem e) ein solches verwendet, welches, bezogen auf das Gesamtgewicht des Reaktionsgemisches, bis zu 0,1 Gew.-% einer unter den Reaktionsbedingungen zu Redoxreaktionen befähigten Verbindung von Elementen der dritten bis fünften Hauptgruppe und/oder der ersten bis achten Nebengruppe des Periodensystems der Elemente und/oder bis zu 10 Gew.-% eines tertiären Amins enthält.

7. Verfahren gemäss Anspruch 1 bis 6, dadurch gekennzeichnet, dass man die Umsetzung im Druckbereich zwischen 5 und 500 bar und im Temperaturbereich zwischen 100°C und 250°C durchführt.

8. Verfahren gemäss Anspruch 1 bis 7, dadurch gekennzeichnet, dass man die Umsetzung, bezogen auf das Gesamtgewicht des Reaktionsgemisches, in Gegenwart von bis zu 80 Gew.-% eines inerten Lösungsmittels durchführt.

9. Verfahren gemäss Anspruch 1 bis 8, dadurch gekennzeichnet, dass man die als Verfahrensprodukte anfallenden Urethane durch Destillation und/oder Filtration von der Katalysatorkomponente e) abtrennt und die Katalysatorkomponente e) wiederverwendet.

## Claims

1. Process for the production of urethanes by reacting

a) primary amines with
b) organic compounds containing at least one hydroxyl group and
c) carbon monoxide in the presence of
d) molecular oxygen as the oxidising agent and
e) a catalyst system containing at least one noble metal and/or noble metal compound of the eighth subgroup of the periodic system of elements,

characterised in that the reaction is carried out in the presence of catalyst systems e) which contain as a further component at least one oxidising quinoid compound and/or at least one compound which can be converted into an oxidising quinoid compound under the reaction conditions.

2. Process according to Claim 1, characterised in that the catalyst system e) used is one which contains as a further component at least one compound of elements of the third to fifth main group and/or of the first to eighth subgroup of the periodic system of elements, which compound is capable of undergoing redox reactions under the reaction conditions.

3. Process according to Claim 1 to 2, characterised in that the catalyst system e) used is one which contains at least one tertiary amine as the further component.

4. Process according to Claim 1 to 3, characterised in that the catalyst system e) used is one which contains, based on the total weight of the reaction mixture, 5 ppm to 100 ppm, calculated as noble metal, and/or of at least one noble metal compound.

5. Process according to Claim 1 to 4, characterised in that the catalyst system e) used is one which contains, based on the total weight of the reaction mixture, 0.1 to 5% by weight of at least one oxidising quinoid compound and/or of at least one compound which can be converted into an oxidising quinoid compound under the reaction conditions.

6. Process according to Claim 1 to 5, characterised in that the catalyst system e) used is one which contains, based on the total weight of the reaction mixture, up to 0.1% by weight of a compound of elements of the third to fifth main group and/or of the first to eighth subgroup of the periodic system of elements, which compound is capable of undergoing redox reactions under the reaction conditions, and/or up to 10% by weight of a tertiary amine.

7. Process according to Claim 1 to 6, characterised in that the reaction is carried out in the pressure range of between 5 and 500 bars and in the temperature range of between 100°C and 250°C.

8. Process according to Claim 1 to 7, characterised in that the reaction is carried out in the presence of up to 80% by weight of an inert solvent, based on the total weight of the reaction mixture.

9. Process according to Claim 1 to 8, characterised in that the urethanes obtained as the process products are separated from the catalyst component e) by distillation and/or filtration and the catalyst component e) is re-used.

## Revendications

1. Procédé de production d'uréthannes par réaction

a) d'amines primaires avec
b) des composés organiques contenant au moins un groupe hydroxyle et
c) de l'oxyde de carbone en présence
d) d'oxygène moléculaire comme agent oxydant et
e) d'un système catalysateur contenant au moins un métal noble et/ou un composé d'un métal noble du sousgroupe VIII du Système Périodique des Eléments,

caractérisé en ce qu'on conduit la réaction en présence de systèmes catalyseurs e) qui contiennent comme autre composant au moins un composé quinonique à action oxydante et/ou au moins un composé qui peut être transformé, dans les conditions réactionnelles, en un composé quinonique à action oxydante.

2. Procédé suivant la revendication 1, caractérisé en ce qu'on utilise comme système catalyseur e) un système qui contient comme autre composant au moins un composé, capable de réactions d'oxydo-réduction dans les conditions réactionnelles, d'éléments des troisième à cinquième groupes principaux et/ou des premier à huitième sous-groupes du Système Périodique des Eléments.

3. Procédé suivant les revendications 1 et 2, caractérisé en ce qu'on utilise comme système catalyseur e) un système qui contient comme autre composant au moins une amine tertiaire.

4. Procédé suivant les revendications 1 à 3, caractérisé en ce qu'on utilise comme système catalyseur e) un système qui contient, par rapport au poids total de la charge réactionnelle, 5 à 100 ppm, exprimés en métal noble, d'au moins un métal noble et/ou d'au moins un composé de métal noble.

5. Procédé suivant les revendications 1 à 4, caractérisé en ce qu'on utilise comme système catalyseur e) un système qui contient, par rapport au poids total de mélange réactionnel, 0,1 à 5% en poids d'au moins un composé quinonique à action oxydante et/ou d'un composé qui peut être transformé en un composé quinonique à action oxydante dans les conditions réactionnelles.

6. Procédé suivant les revendications 1 à 5, caractérisé en ce qu'on utilise comme système catalyseur e) un système qui contient, par rapport au poids total de mélange réactionnel, jusqu'à 0,1% en poids d'un composé, apte à des réactions d'oxydo-réduction dans les conditions réactionnelles, d'éléments des troisième à cinquième groupes principaux et/ou des premier à huitième sous-groupes du Système Périodique des Eléments et/ou jusqu'à 10% en poids d'une amine tertiaire.

7. Procédé suivant les revendications 1 à 6, caractérisé en ce qu'on conduit la réaction dans l'intervalle de pression de 5 à 500 bars et dans l'intervalle de température de 100 à 250°C.

8. Procédé suivant les revendications 1 à 7, caractérisé en ce qu'on conduit la réaction, par rapport au poids total du mélange réactionnel, en présence d'une quantité allant jusqu'à 80% en poids d'un solvant inerte.

9. Procédé suivant les revendications 1 à 8, caractérisé en ce qu'on sépare du composant catalyseur e) les uréthannes obtenus comme produits du procédé par distillation et/ou par filtration et on réutilise le composant catalyseur e).